# EUROPÄISCHE PATENTSCHRIFT

(11) **EP 0 710 271 B1**
(45) Veröffentlichungstag und Bekanntmachung des Hinweises auf die Patenterteilung: **05.02.1997**
(21) Anmeldenummer: 94922243.4
(22) Anmeldetag: 29.06.1994
(51) Int. Cl.: C10L 1/22, C08G 65/32, C07C 237/06, C07C 237/08, C07C 237/10

(54) **UMSETZUNGSPRODUKTE VON AMINOALKYLENCARBONSÄUREN SOWIE ERDÖLMITTELDESTILLATE, DIE DIESE ENTHALTEN**
REACTION PRODUCTS OF AMINO ALKYLENE CARBOXYLIC ACIDS AND PETROLEUM MIDDLE DISTILLATES CONTAINING THEM
PRODUITS DE REACTION D'ACIDES CARBOXYLIQUES D'AMINOALKYLENE ET DISTILLATS MOYENS DE PETROLE LES CONTENANT

(30) Priorität: 21.07.1993 DE 4324394
(43) Veröffentlichungstag der Anmeldung: 08.05.1996
(73) Patentinhaber: BASF AKTIENGESELLSCHAFT, 67056 Ludwigshafen (DE)
(72) Erfinder: DRALLE-VOSS, Gabriele, D-64297 Darmstadt (DE); OPPENLÄNDER, Knut, D-67061 Ludwigshafen (DE); BARTHOLD, Klaus, D-68259 Mannheim (DE); WENDEROTH, Bernd, D-69488 Birkenau (DE); KASEL, Wolfgang, D-69226 Nu loch (DE)
(86) Internationale Anmeldenummer: EP9402122
(87) Internationale Veröffentlichungsnummer: WO9503378

(56) Entgegenhaltungen:
- EP-A- 0 188 786
- EP-A- 0 277 345
- EP-A- 0 301 448
- EP-A- 0 398 101
- EP-A- 0 464 489
- WO-A-92/09673
- US-A- 3 024 277

## Beschreibung

Die Erfindung betrifft als Paraffindispergatoren geeignete Umsetzungsprodukte von Aminoalkylencarbonsäuren sowie deren Verwendung und Erdölmitteldestillatzusammensetzungen auf der Basis eines Kohlenwasserstoffgemisches, die diese Umsetzungsprodukte enthalten.

Mitteldestillate wie z.B. Gasöle, Dieselöle oder Heizöle, die durch Destillation aus Erdölen gewonnen werden, haben je nach Herkunft des Rohöls unterschiedliche Gehalte an Paraffinen. Bei tieferen Temperaturen kommt es zur Ausscheidung fester Paraffine (Trübungspunkt oder Cloud Point, CP). Bei weiterer Abkühlung bilden die plättchenförmigen n-Paraffinkristalle eine "Kartenhausstruktur" und das Mitteldestillat stockt, obwohl der überwiegende Teil des Mitteldestillates noch flüssig ist. Durch die ausgefallenen n-Paraffine im Temperaturgebiet zwischen Trübungspunkt und Stockpunkt (bzw. Pour Point) wird die Fließfähigkeit der Erdölmitteldestillate erheblich beeinträchtigt. Die Paraffine verstopfen Filter und verursachen ungleichmäßige oder völlig unterbrochene Kraftstoffzufuhr zu den Verbrennungsaggregaten.

Es ist seit langem bekannt, daß durch geeignete Zusätze das Kristallwachstum der Paraffine in den Erdölmitteldestillate modifiziert werden kann. Gut wirksame Additive verhindern einerseits, daß Mitteldestillate derartige Kartenhaus-Strukturen ausbilden und bei Temperaturen wenige Grad Celsius unterhalb der Temperatur, bei welcher die ersten Paraffinkristalle auskristallisieren, bereits fest werden und andererseits feine, gut kristallisierte, separate Paraffinkristalle bilden, welche Filter in Kraftfahrzeugen und Heizungsanlagen passieren oder zumindest einen für den flüssigen Teil der Mitteldestillate durchlässigen Filterkuchen bilden, so daß ein störungsfreier Betrieb sichergestellt ist.

Ein Nachteil dieses Standes der Technik beruht darauf, daß die ausgefallenen Paraffinkristalle aufgrund ihrer gegenüber dem flüssigen Teil höheren Dichte dazu neigen, sich beim Lagern mehr und mehr am Boden des Behälters abzusetzen. Dadurch bildet sich eine im oberen Behälterteil homogene paraffinarme Phase und am Boden eine zweiphasige paraffinreiche Schicht. Da sowohl in Fahrzeugtanks als auch in Lager- oder Liefertanks der Mineralölhändler der Abzug des Mitteldestallates meist wenig oberhalb des Behälterbodens erfolgt, besteht die Gefahr, daß die hohe Konzentration an festen Paraffinen zu Verstopfungen von Filtern und Dosiereinrichtungen führt. Diese Gefahr wird umso größer, je weiter die Lagertemperatur die Ausscheidungstemperatur der Paraffine (Trübungspunkt) unterschreitet, da die ausgeschiedene Paraffinmenge eine Funktion der Temperatur darstellt und mit sinkender Temperatur ansteigt.

Bei den Paraffinkristallmodifikatoren, den sog. Fließverbesserern, handelt es sich um Polymere, die durch Co-Kristallisation (Interaktion) das Kristallwachstum der n-Paraffine verändern. Dabei werden die Fließeigenschaften des Mitteldestillats bei niedrigeren Temperaturen positiv beeinflußt. Die Wirksamkeit der Fließverbesserer wird nach DIN 51428 indirekt durch Messung des "Cold Filter Plugging Point" ("CFPP") ausgedrückt.

Als Kältefließverbesserer werden an sich bekannte Ethylencopolymerisate, vor allem Copolymere von Ethylen und ungesättigten Estern, verwendet. DE 11 47 799 und DE 19 14 756 beschreiben beispielsweise Copolymerisate des Ethylens mit Vinylacetat, mit einem Gehalt von 25 bis 45 Gew.-% Vinylacetat oder Vinylpropionat und einem Molekulargewicht von 500 bis 5000.

Weiterhin ist aus GB 2 095 698 bekannt, Mitteldestillaten eine Kombination aus den genannten Copolymeren mit Amiden aus langkettigen Aminen und aromatischen oder cycloaliphatischen Carbonsäuren zuzusetzen.

Bezüglich der Dispergiereigenschaften der ausgeschiedenen Paraffine befriedigen diese Mischungen aber noch nicht. Es ist daher erforderlich, daß die zugesetzten Additive auch die Dispergierung der ausgeschiedenen Paraffine bewirken.

Aus der EP-A 398 101 sind Umsetzungsprodukte von Aminoalkylenpolycarbonsäuren mit langkettigen sekundären Aminen, die vollständig mit dem Amin zum Amid oder Ammoniumsalz umgesetzt worden sind, als Paraffindispergatoren bekannt.

Die noch nicht veröffentlichte deutsche Patentanmeldung P 4237662.9 lehrt Umsetzungsprodukte von Aminoalkylenpolycarbonsäuren mit langkettigen sekundären Aminen, in denen noch freie Carboxylgruppen oder Alkalimetall- oder Erdalkalimetallcarboxylatgruppen vorliegen.

Diese Paraffindispergatoren werden im allgemeinen in Form einer konzentrierten Lösung in öllöslichen Lösungsmitteln wie hochsiedenden Petrolethern oder Aromatengemischen in den Handel gebracht. Diese Dispergatoren können aber in konzentrierter Lösung einen so hohen Stockpunkt haben, daß ihre Handhabung beim Umfüllen oder Dosieren erschwert ist.

Es bestand daher die Aufgabe, Paraffindispergatoren für Erdölmitteldestillate zu finden, die neben einer guten Dispergierwirkung einen niedrigen Stockpunkt in konzentrierter Lösung aufweisen.

Demgemäß wurden die Verbindungen der Formeln I und II gefunden 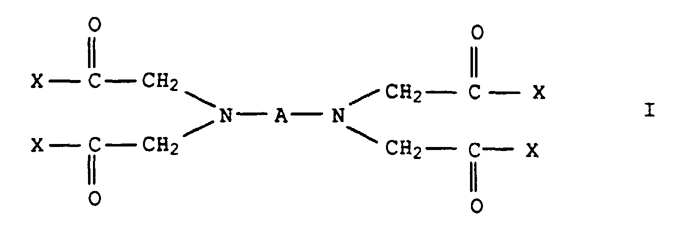 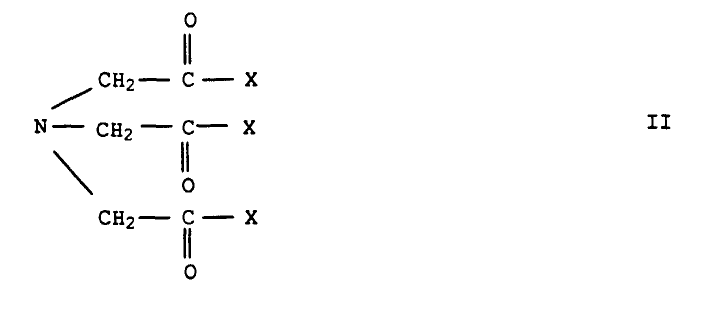 worin bedeuten
- A: einen geradkettigen oder verzweigten Alkylenrest mit 2 bis 6 Kohlenstoffatomen oder den Rest der Formel III 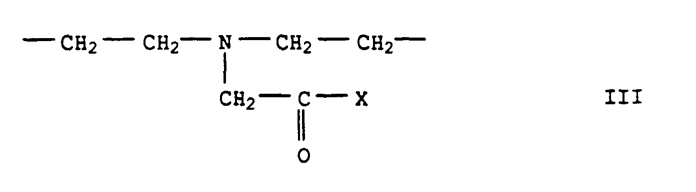
- X: die Reste 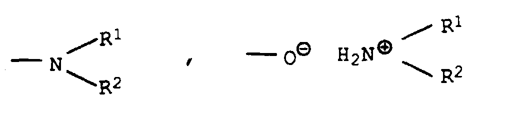 und/oder einen Rest der Formel IV 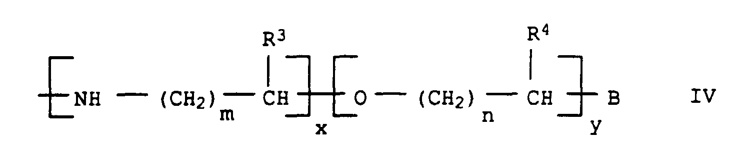 wobei die Variablen folgende Bedeutung haben:
R¹ Wasserstoff, geradkettiger aliphatischer C₁₀-C₃₀-Rest;
R² geradkettiger aliphatischer C₁₀-C₃₀-Rest;
R³,R⁴ Wasserstoff, Methyl, Ethyl;
m,n 1 bis 5;
x 0 bis 3;
y 1 bis 100;
B Hydroxy, C₁-C₃₀-Alkoxy, gegebenenfalls ein- bis dreifach durch einen C₁-C₁₈-Alkylrest substituiertes Phenoxy oder Naphthoxy, C₁-C₃₀-Alkycarboxy oder Alkenylcarboxy, mit geradkettigem aliphatischen C₁-C₃₀-Resten monosubstituiertes Amino, mit geradkettigen aliphatischen C₁-C₃₀-Resten disubstituiertes Amino, C₁-C₃₀-Alkylamido oder Alkenylamido, eine Gruppe der Formel V 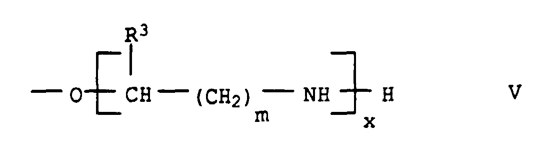 in der die Variablen die oben angegebene Bedeutung haben, mit der Maßgabe, daß die folgenden beiden Bedingungen erfüllt sind, wonach X
a) mindestens eine 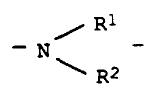 Gruppe und
b) mindestens eine Gruppe der Formel IV bedeutet.

Weiterhin wurde die Verwendung der Verbindungen I und II als Paraffindispergatoren gefunden sowie Erdölmitteldestillate, die diese Verbindungen enthalten.

Die Umsetzung von Ethylentriamintetraessigsäure, Diethylentriaminpentaessigsäure bzw. Nitrilotriessigsäure mit Aminen HNR¹R² führt zu Verbindungen der Formel I bzw. II, in denen X für -NR¹R² oder das entsprechende Ammoniumslaz -O^{⊖}H₂N^{⊕}R¹R² steht. Die Reste R¹ und R² stehen für geradkettige aliphatische C₁₀-C₃₀-Reste, vorzugsweise C₁₄-C₂₂-Reste, R¹ steht weiterhin für Wasserstoff. Besonders bevorzugt stehen sowohl R¹ als auch R² für einen geradkettigen aliphatischen C₁₀-C₃₀-Rest, insbesondere für einen C₁₄-C₂₂-Rest, d.h. bei den zur Herstellung der Verbindungen I und II eingesetzten Aminen handelt es sich um sekundäre Amine. Als sekundäre Amine seien im einzelnen Dioleylamin, Ditalgfettamin, Dipalmitinamin, Dikokosfettamin und Dibehenylamin und vorzugsweise Distearylamin oder hydriertes Ditalgfettamin (letzteres mit jeweils 16 bis 18 C-Atomen) genannt.

Die Gruppen der Formel IV werden durch Umsetzung der Verbindungen VI 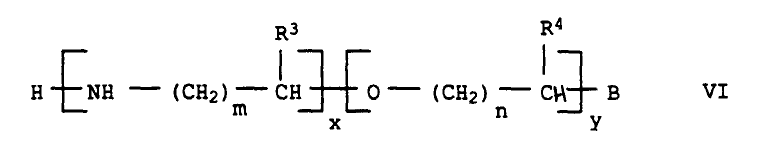 mit den genannten Polycarbonsäuren in die Paraffindispergatoren I und II eingebaut.

Es handelt sich bei den Verbindungen VI um Polyoxyalkylenderivate. Die Reste R³ und R⁴ stehen für Wasserstoff, Methyl und Ethyl. Die Variablen m und n stehen für 1 bis 5. Die Verbindungen sind also durch Polymerisation von Alkylenoxiden wie Ethylenoxid, Propylenoxid und Butylenoxid oder durch polymerisierende Ringöffnung von Tetrahydrofuran zugänglich. Die Variablen x und y geben den Polymerisationsgrad an. X steht für 0 bis 5, vorzugsweise 0, und y steht für 1 bis 100, vorzugsweise 1 bis 20. Steht B für Hydroxy, handelt es sich bei den Verbindungen VI um Polyalkylenglykole wie Polyethylenglykol, Polypropylenglykol, Polytetrahydrofuranverbindungen oder gemischte Copolymere aus z.B. Ethylenoxid und Propylenoxid. Im allgemeinen liegt das mittlere Molekulargewicht der Glykole bei 200 bis 4000.

Weiterhin kommen alkoxylierte Alkohole als Verbindungen VI in Betracht. B steht in diesem Fall für geradkettige oder verzweigte C₁-C₃₀-Alkoxyreste, vorzugsweise für C₈-C₂₂-Reste. Sie leiten sich von den entsprechenden Alkoholen wie Isotridecanol, Isodecanol, Decanol, Talgfettalkohol und Stearylalkohol ab.

Die Verbindungen VI umfassen außerdem alkoxylierte Phenole und Naphthole, welche gegebenenfalls ein- bis dreifach durch C₁-C₁₈-Alkylreste substituiert sind. Die Gruppe B leitet sich bei dieser Verbindungsklasse beispielsweise von p-Kresol, Di-tert.- butylphenol, Isooctylphenol, Isononylphenol und β-Naphthol ab.

Bei Verbindung VI handelt es sich um alkoxylierte Carbonsäuren, wenn B für C₁-C₃₀-Alkylcarboxy oder -Alkenylcarboxy steht. Bei den Carbonsäuren handelt es sich vorzugsweise um geradkettige Säuren mit 8 bis 22 Kohlenstoffatomen wie Stearinsäure, Laurinsäure, Ölsäure, Behensäure, Talgfettsäure, 2-Ethylhexansäure und Isononansäure.

Weiter kann die Gruppe B für Aminoreste stehen, die einfach oder zweifach durch aliphatische C₁-C₃₀-Reste, vorzugsweise C₁₀-C₂₂-Reste, substituiert sind. Diese Aminoreste leiten sich von Aminen wie Behenylamin, Distearylamin, Ditalgfettamin oder hydriertem Ditalgfettamin ab.

Schließlich kann die Gruppe B für C₁-C₃₀-Alkylamidoreste oder -Alkenylamidoreste wie die Reste von Stearylsäureamid und Ölsäureamid stehen.

Ist x in den Verbindungen VI größer als Null, liegen alle genannten Polyoxyalkylenverbindungen aminiert vor. Diese Aminierung kann in bekannter Weise durch aminierende Hydrierung oder Aminopropylierung der entsprechenden Hydroxyverbindungen erfolgen. Werden Polyglykole an beiden Kettenenden aminiert, erhält man Verbindungen VI, in denen der Rest B für die Formel V steht.

Bevorzugt sind solche Verbindungen I, die drei Reste -NR¹R² und einen Rest der Formel IV tragen.

Zur Herstellung der Verbindungen I und II können die Polycarbonsäuren, mindestens 1 Äquivalent der Amine HNR¹R² und mindestens 1 Äquivalent der Verbindungen VI vermischt und auf Temperaturen von 100 bis 200°C erhitzt werden, wobei das Reaktionswasser kontinuierlich abdestilliert werden kann. Vorzugsweise nimmt man die Reaktion in Gegenwart katalytischer Mengen einer Säure wie Toluolsulfonsäure vor.

Die erfindungsgemäßen Verbindungen können als Paraffindispergatoren verwendet werden. Sie können Erdölmitteldestillaten zugesetzt werden, bevorzugt solchen mit einem Siedebeginn über 160°C und einem Siedeende unter 420°C.

Die Verbindungen der Formeln I bzw. II werden Erdölmitteldestillatzusammensetzungen in der Regel in Mengen von 25 bis 1000 ppm, bevorzugt 50 bis 500 ppm, zugesetzt.

Üblicherweise enthalten die Mitteldestillate bereits an sich bekannte Fließverbesserer, die in der Patentliteratur eingehend beschrieben sind, z.B. in DE 19 14 756 und EP-A 486 836 (Ethylen-Vinylester-Copolymerisate und deren Mischung mit anderen Copolymeren), EP 214 876 (α-Olefin-Maleinsäureanhydridester) oder EP 155 807 (Alkylfumarat-Vinylacetat-Copolymere).

Es kommen jedoch auch gleichermaßen Terpolymere in Betracht, die neben Ethylen und Vinylestern oder Acrylestern noch weitere Comonomere enthalten. Das Molekulargewicht dieser Fließverbesserer beträgt in der Regel 500 bis 5000, vorzugsweise 1000 bis 3000. Auch Mischungen verschiedener Fließverbesserer kommen in Betracht.

5 Weiterhin können die Mitteldestillate gegebenenfalls einen Leitfähigkeitsverbesserer enthalten, wie sie beispielsweise in DE-A 21 16 556 beschrieben sind. Die Verbindungen I und II besitzen neben guten dispergierenden Eigenschaften den Vorteil, in großer Konzentration in öllöslichen Lösungsmitteln einen niedrigen Stockpunkt zu haben.

### Beispiele

### A) Herstellung der Aminoalkylenpolycarbonsäureamide und -esteramide (Verbindung I)

Allgemeine Herstellvorschrift für die Paraffindispergatoren PD1-PD8:

151,5 g (0,3 mol) Distearylamin, 0,1 mol Verbindung VI und ca. 1,1 g p-Toluolsulfonsäure wurden vorgelegt und aufgeschmolzen. Bei 100-110°C wurden 29,5 g (0,1 mol) Ethylendiamintetraessigsäure zugegeben. Unter Stickstoffatmosphäre wurde das Reaktionsgemisch auf 190°C erhitzt und solange bei dieser Temperatur kondensiert, bis die Säurezahl unter 10 mg KOH/g gesunken war. Das Reaktionswasser destillierte dabei vollständig ab. Nach Filtration erhielt man einen braunen wachsartigen Feststoff.

Für alle Verbindungen war im IR die Amidbande bei 1650 cm⁻¹ charakteristisch. Die Amidester zeigten zusätzlich eine Esterbande bei 1730 cm⁻¹.

PD1: Umsetzungsprodukt aus Ethylendiamintetraessigsäure, Distearylamin und ethoxiliertem Isononylphenol (Molgewicht: 490 g/mol, Ethoxylierungsgrad: 6) im molaren Verhältnis 1:3:1.

PD2: Umsetzungsprodukt aus Ethylendiamintetraessigsäure, Distearylamin und ethoxiliertem Isononylphenol (Molgewicht: 578 g/mol, Ethoxylierungsgrad: 8) im molaren Verhältnis 1:3:1.

PD3: Umsetzungsprodukt aus Ethylendiamintetraessigsäure, Distearylamin und Polyethylenglykol mit Molgewicht 200 g/mol im molaren Verhältnis 1:3:1.

PD4: Umsetzungsprodukt aus Ethylendiamintetraessigsäure, Distearylamin und Polyethylenglykol mit Molgewicht 300 g/mol im molaren Verhältnis 1:3:1.

PD5: Umsetzungsprodukt aus Ethylendiamintetraessigsäure, Distearylamin und N-(2-Hydroxy-1-methylethyl)-distearylamin im molaren Verhältnis 1:3:1.

PD6: Umsetzungsprodukt aus Ethylendiamintetraessigsäure, Distearylamin und Polypropylenglykoletherdiamin mit Molgewicht 2000 g/mol im molaren Verhältnis 1:3:1.

PD7: Umsetzungsprodukt aus Ethylendiamintetraessigsäue, Distearylamin und N-(Hydroxyethyl)distearylamin im molaren Verhältnis 1:3:1.

PD8: Umsetzungsprodukt aus Ethylendiamintetraessigsäure, Distearylamin und propoxiliertem Distearylamin mit Propoxilierungsgrad 5 im molaren Verhältnis 1:3:1.

### B) Prüfung der Erdölmitteldestillatzusammensetzungen

Folgende Erdölmitteldestillatzusammensetzungen wurden geprüft:

### 1) als Paraffindispergator PD:

PD 1 - PD 8 Ethylendiamintetraessigsäure-Derivate; als Vergleich PD 9 (Ethylendiamintetraessigsäureamid A1 aus EP-A 398 101)

### 2) als Fließverbesserer F1:

Fl (A) Ethylen/Vinylpropionat (mit ca. 40 Gew.-% Vinylpropionat) mit einem mittleren Molekulargewicht von ca. 2500 (Fl (A) aus EP-A 398 101)

### 3) als Leitfähigkeitsverbesserer LV:

LV (E) aus EP-A 398 101

Als Mitteldestillate wurden für die folgenden Dispergierversuche Dieselkraftstoffe in handelsüblicher deutscher Raffineriequalität verwendet; sie werden als DK 1, DK 2, DK 3 bezeichnet:

| | DK 1 | DK 2 | DK 3 |
|---|---|---|---|
| Trübungspunkt CP (°C) | -8 | -8 | -7 |
| CFPP (°C) | -13 | -12 | -10 |
| Dichte b. 20°C (g/ml) | 0.827 | 0.831 | 0.829 |
| Siedeanfang (°C) | 165 | 175 | 183 |
| 20 % Siedepunkt (°C) | 210 | 223 | 211 |
| 90 % Siedepunkt (°C) | 318 | 314 | 317 |
| Siedeende (°C) | 358 | 352 | 364 |

### Beschreibung der Testmethode:

Die Mitteldestillate wurden mit unterschiedlichen Mengen Paraffindispergatoren PD 1 - PD 8 bzw. PD 9 (jeweils als 50 %-ige Lösung in Solvesso^{®} 150 (hochsiedendes Aromatengemisch mit einem Siedebereich von 186 bis 206°C der Firma Esso), Fließverbesserer F1 und Leitfähigkeitsverbesserer LV bei 40°C unter Rühren additiviert und anschließend auf Raumtemperatur abgekühlt.

Die additivierten Mitteldestillate wurden in 100 ml-Meßzylindern für 16 oder 20 Stunden in einem Kälteschrank bei -13°C oder bei -18°C gelagert. Anschließend wurde visuell das Volumen der sedimentierten Paraffinphase (Vol-%) und das Aussehen der Ölphase beurteilt.

In den Tabellen I - III sind die Ergebnisse aufgeführt. Es wird ersichtlich, daß die Verbindungen PD 1 - PD 8 in den Mitteldestillaten eine vergleichbar gute Dispergierwirkung zeigen wie die Vergleichsverbindung PD 9.

Der Vorteil der erfindungsgemäßen Verbindungen liegt darin, daß deren 50%ige Lösung in einem Lösungsmittel im Vergleich zur Lösung von PD 9 einen niedrigeren Stockpunkt haben (siehe Tabelle IV), was die Handhabbarkeit in der Praxis wesentlich erleichtert.

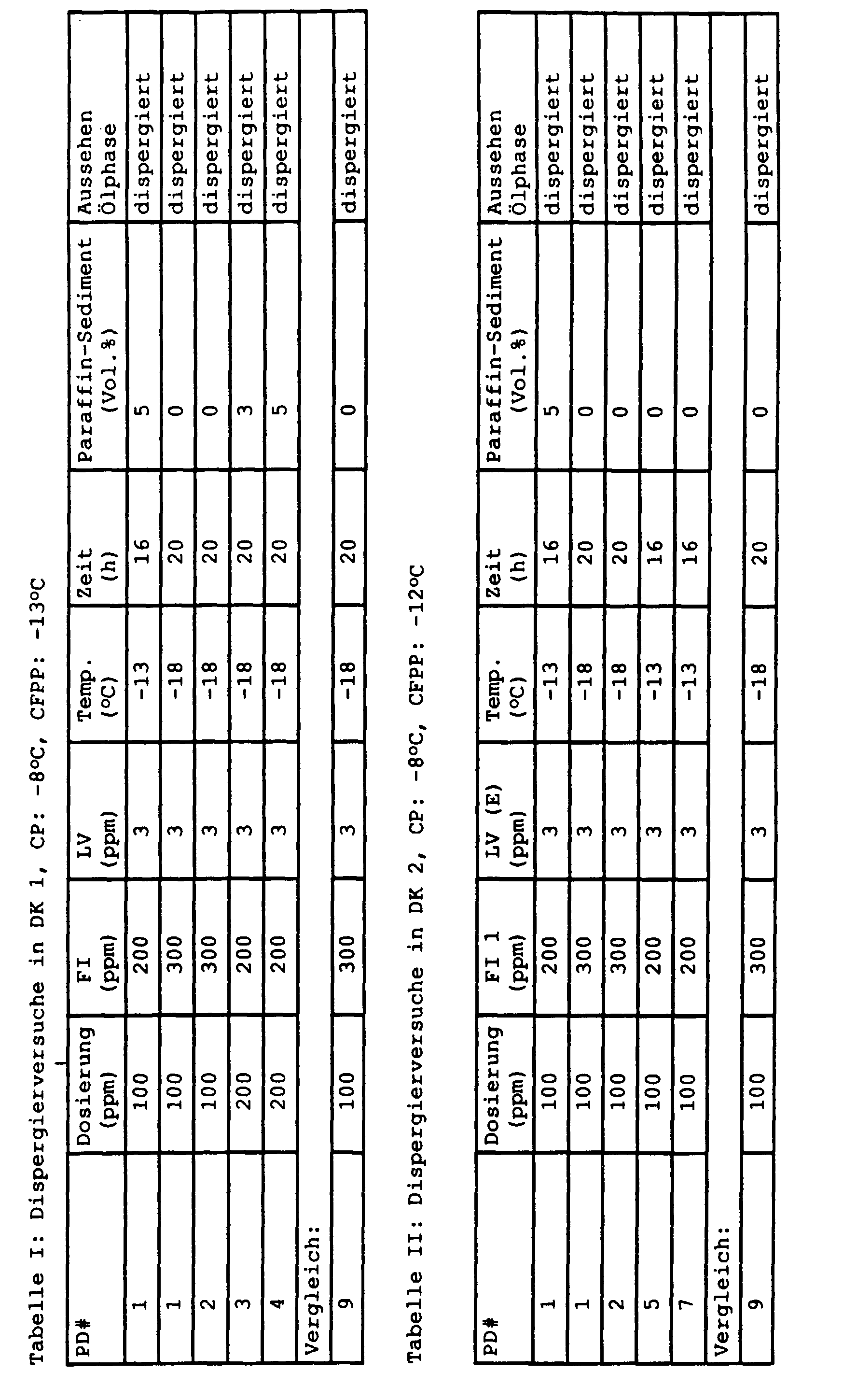

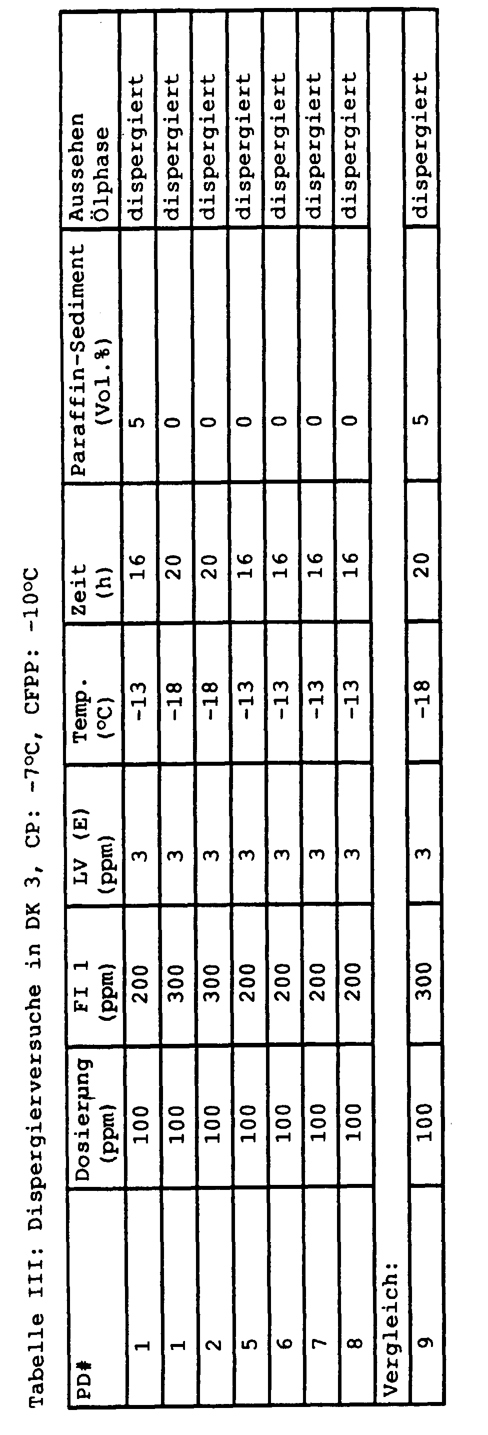

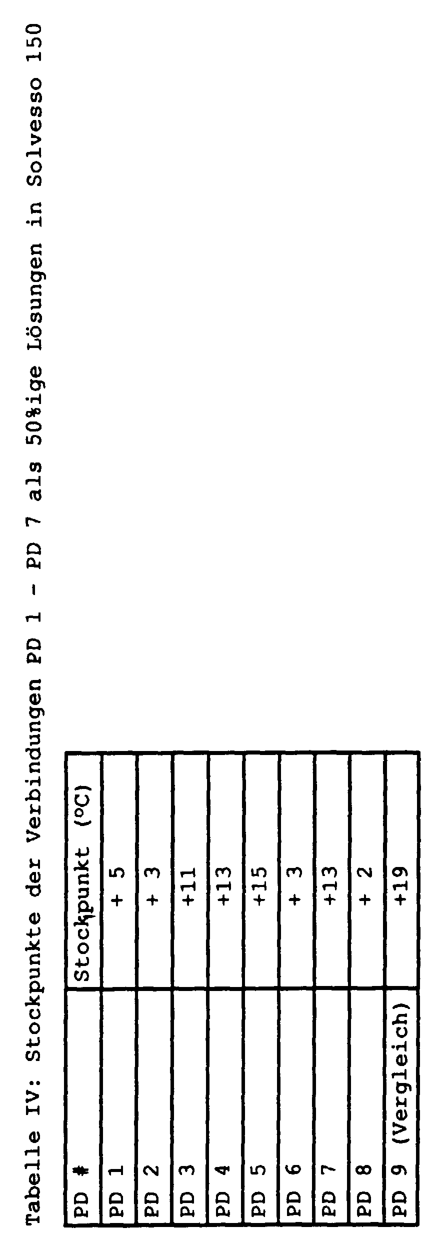

## Patentansprüche

1. Als Paraffindispergatoren für Erdölmitteldestillate geeignete Umsetzungsprodukte von Aminoalkylencarbonsäuren der allgemeinen Formeln I und II 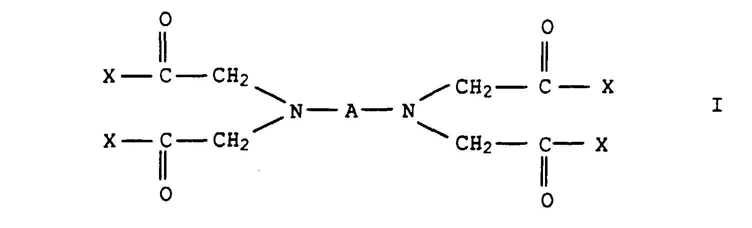 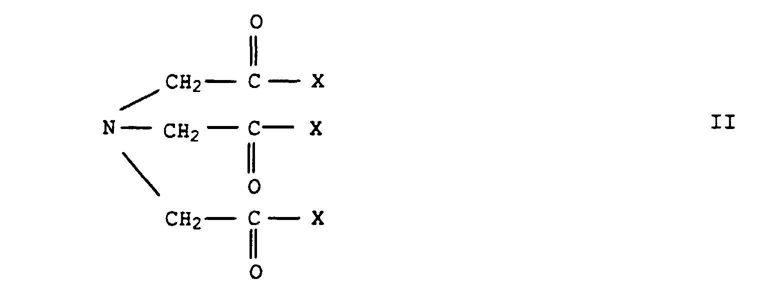 worin bedeuten
A einen geradkettigen oder verzweigten Alkylenrest mit 2 bis 6 Kohlenstoffatomen oder den Rest der Formel III 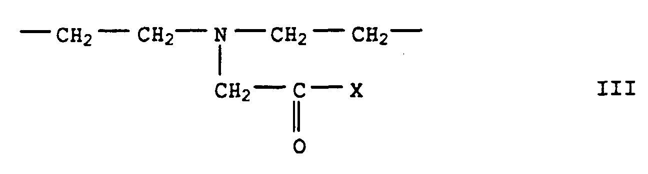
X die Reste 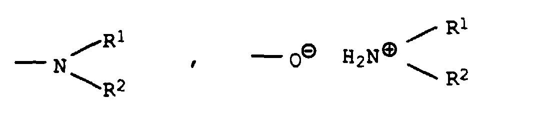 und/oder einen Rest der Formel IV 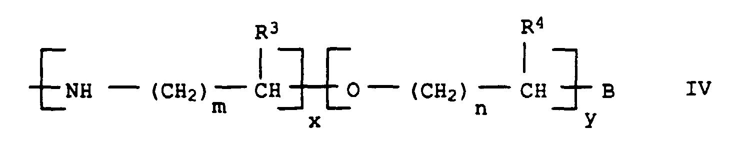 wobei die Variablen folgende Bedeutung haben:
R¹ Wasserstoff, geradkettiger aliphatischer C₁₀-C₃₀-Rest;
R² geradkettiger aliphatischer C₁₀-C₃₀-Rest;
R³,R⁴ Wasserstoff, Methyl, Ethyl;
m,n 1 bis 5;
x 0 bis 3;
y 1 bis 100;
B Hydroxy, C₁-C₃₀-Alkoxy, gegebenenfalls ein- bis dreifach durch einen C₁-C₁₈-Alkylrest substituiertes Phenoxy oder Naphthoxy, C₁-C₃₀-Alkylcarboxy oder -Alkenylcarboxy, mit geradkettigen aliphatischen C₁-C₃₀-Resten monosubstituiertes Amino, mit geradkettigen aliphatischen C₁-C₃₀-Resten disubstituiertes Amino, C₁-C₃₀-Alkylamido oder Alkenylamido, eine Gruppe der Formel V 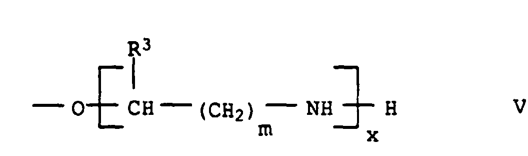 in der die Variablen die oben angegebene Bedeutung haben, mit der Maßgabe, daß die folgenden beiden Bedingungen erfüllt sind, wonach X
a) mindestens eine 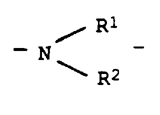 Gruppe und
b) mindestens eine Gruppe der Formel IV bedeutet.

2. Verbindungen nach Anspruch 1, in denen R¹ Wasserstoff oder einen geradkettigen aliphatischen Rest mit 14 bis 22 Kohlenstoffatomen und R² einen geradkettigen aliphatischen Rest mit 14 bis 22 Kohlenstoffatomen bedeutet.

3. Verbindungen der Formel I nach Anspruch 1,
in denen X dreimal für den Rest 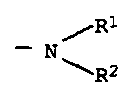 und einmal für einen Rest der Formel IV steht.

4. Verwendung der Verbindungen gemäß den Ansprüchen 1 bis 3 als Paraffindispergatoren in Erdölmitteldestillaten.

5. Erdölmitteldestillatzusammensetzungen auf Basis eines Kohlenwasserstoffgemisches, dessen Siedebeginn über 160°C und dessen Siedeende unter 420°C liegt, enthaltend eine als Paraffindispergator wirksame Menge der Verbindungen I und/oder II gemäß den Ansprüchen 1 bis 3.

## Claims

1. A reaction product suitable as a paraffin dispersant for mineral oil middle distillates and obtained from aminoalkylenecarboxylic acids of the general formulae I and II 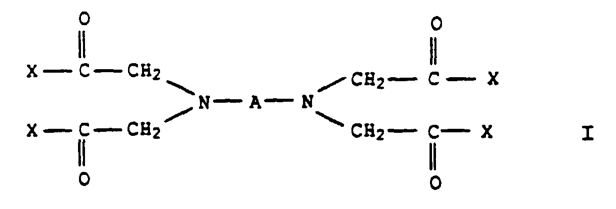 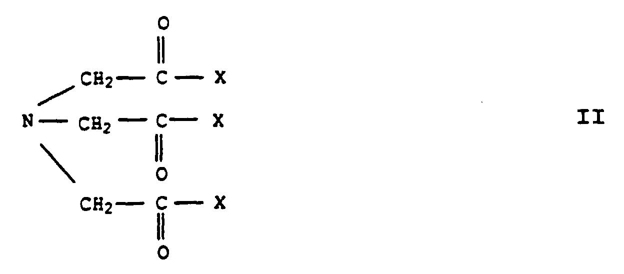 where
A is straight-chain or branched alkylene of 2 to 6 carbon atoms or a radical of the formula III 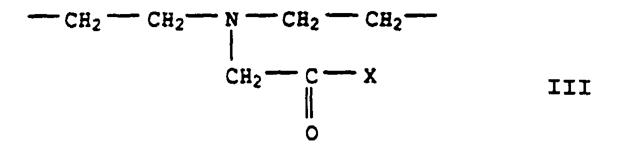
X is 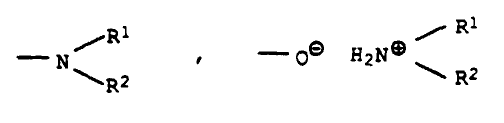 and/or a radical of the formula IV 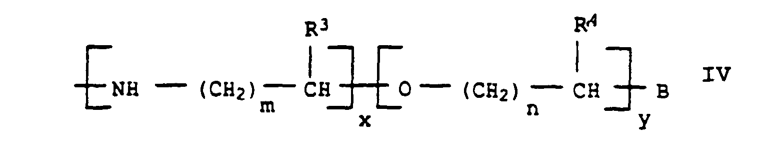 where R¹ is hydrogen or a straight-chain aliphatic C₁₀-C₃₀ radical,
R² is a straight-chain aliphatic C₁₀-C₃₀ radical,
R³ and R⁴ are each hydrogen, methyl or ethyl,
m and n are each from 1 to 5,
x is from 0 to 3,
y is from 1 to 100 and
B is hydroxyl or C₁-C₃₀-alkoxy or is naphthyloxy or phenoxy which is unsubstituted or monosubstituted to trisubstituted by C₁-C₁₈-alkyl, or is C₁-C₃₀-alkylcarboxy or C₁-C₃₀-alkenylcarboxy, or is amino which is monosubstituted by a straight-chain aliphatic C₁-C₃₀ radical, or is amino which is disubstituted by straight-chain aliphatic C₁-C₃₀ radicals, or is C₁-C₃₀-alkylamido or alkenylamido or a group of the formula V 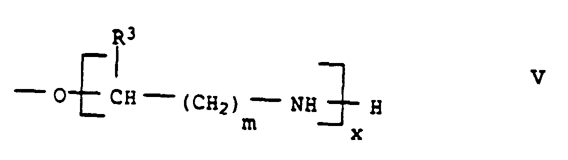 where the variables have the abovementioned meanings, with the proviso that x is
a) at least one 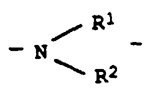 group and
b) at least one group of the formula IV, both conditions being fulfilled.

2. A compound as claimed in claim 1, in which R¹ is hydrogen or a straight-chain aliphatic radical of 14 to 22 carbon atoms and R² is a straight-chain aliphatic radical of 14 to 22 carbon atoms.

3. A compound of the formula I as claimed in claim 1, in which three radicals X are each 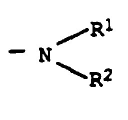 and one radical X is a radical of the formula IV.

4. Use of a compound as claimed in any of claims 1 to 3 as a paraffin dispersant in mineral oil middle distillates.

5. A mineral oil middle distillate composition based on a hydrocarbon mixture whose initial boiling point is above 160°C and whose final boiling point is below 420°C, containing an amount, effective as the paraffin dispersant, of a compound I and/or II as claimed in any of claims 1 to 3.

## Revendications

1. Produits de réaction d'acides aminoalkylènecarboxyliques de formules générales I et II, convenant comme dispersants de paraffines pour distillats moyens de pétrole, 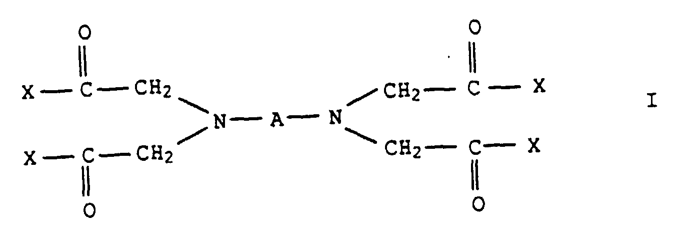 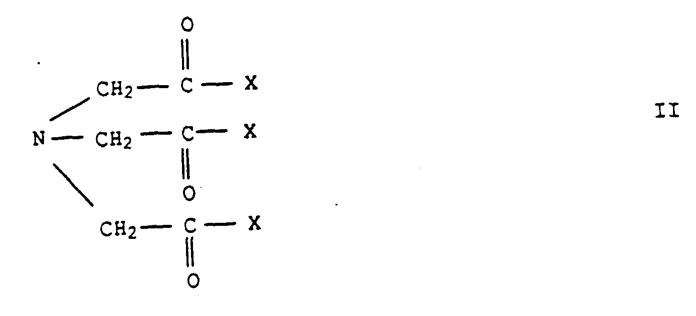 dans lesquelles
A représente un reste alkylène à chaîne droite ou ramifiée à 2-6 atomes de carbone ou le reste de formule III 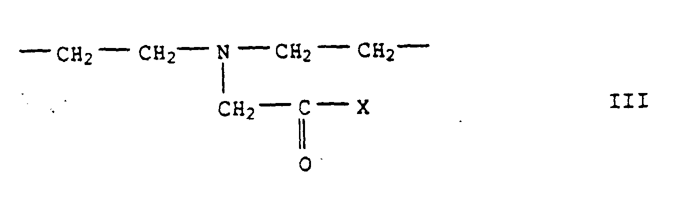
X représente les restes 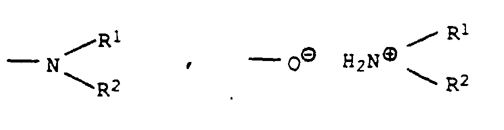 et/ou un reste de formule IV 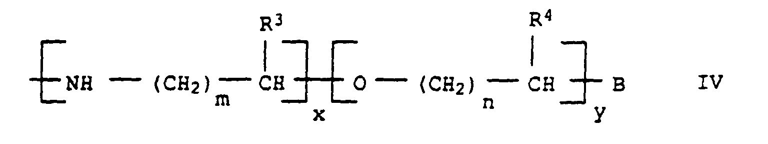 les variables ayant les significations suivantes:
R¹ Atome d'hydrogène, reste aliphatique en C₁₀-C₃₀ à chaîne droite;
R² Reste aliphatique en C₁₀-C₃₀ à chaîne droite;
R³,R⁴ Atomes d'hydrogène, restes méthyle, éthyle;
m,n 1 à 5;
x 0 à 3;
y 1 à 100;
B Reste hydroxy, alcoxy en C₁-C_{30,} phénoxy ou naphtyloxy éventuellement substitué une à trois fois par un reste alkyle en C₁-C₁₈, (alkyl en C₁-C₃₀)carboxy ou (alcényl en C₁-C₃₀)carboxy, amino monosubstitué par des restes aliphatiques en C₁-C₃₀ à chaîne droite, amino bisubstitué par des restes aliphatiques en C₁-C₃₀ à chaîne droite, (alkyl en C₁-C₃₀)amido ou (alcényl en C₁-C₃₀)amido, groupement de formule V 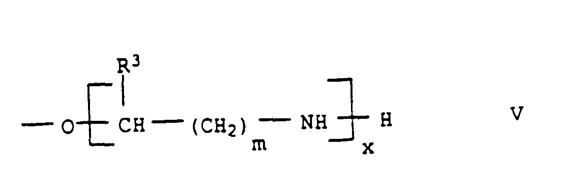 dans laquelle les variables ont les significations données ci-dessus,
étant spécifié que les deux conditions suivantes sont satisfaites, à savoir que X représente
a) au moins une fois un groupement 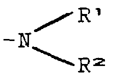 et
b) au moins une fois un groupement de formule IV.

2. Composés selon la revendication 1, dans lesquels R¹ représente un atome d'hydrogène ou un reste aliphatique à chaîne droite à 14-22 atomes de carbone et R² représente un reste aliphatique à chaîne droite à 14-22 atomes de carbone.

3. Composés de formule I selon la revendication 1, dans lesquels X est mis trois fois pour le reste 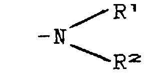 et une fois pour un reste de formule IV.

4. Utilisation des composés selon l'une quelconque des revendications 1 à 3 comme dispersants de paraffines dans des distillats moyens de pétrole.

5. Compositions de distillats moyens de pétrole à base d'un mélange d'hydrocarbures dont le début d'ébullition se situe au-dessus de 160°C et dont la fin d'ébullition se situe au-dessous de 420°C, contenant des composés I et/ou II selon l'une quelconque des revendications 1 à 3 en une quantité efficace en tant que dispersant de paraffines.
